# FASCICULE DE BREVET EUROPEEN

(11) **EP 3 420 122 B1**
(45) Date de publication et mention de la délivrance du brevet: **16.12.2020**
(21) Numéro de dépôt: 17704797.4
(22) Date de dépôt: 17.02.2017
(51) Int. Cl.: C25B 1/10, C25B 9/10, G01N 33/00

(54) **DISPOSITIF POUR LA PRODUCTION D'HYDROGÈNE GAZEUX**
VORRICHTUNG ZUR HERSTELLUNG VON GASFÖRMIGEM WASSERSTOFF
DEVICE FOR THE PRODUCTION OF GASEOUS HYDROGEN

(30) Priorité: 23.02.2016 BE 201605124
(43) Date de publication de la demande: 02.01.2019
(73) Titulaire: H2Win S.A., 1400 Nivelles (BE)
(72) Inventeur: LORGE, Philippe, 1400 Nivelles (BE); REMACLE, Claire, 4600 Visé (BE); GERIN, Stéphanie, 4530 Villers le Bouillet (BE); JOB, Nathalie, 4051 Vaux-sous-Chèvremont (BE); FRANCK, Fabrice, 4000 Liège (BE); CALDARELLA, Giuseppe, 4430 Ans (BE); GHYSELS, Bart, 1020 Laeken (BE); GODAUX, Damien, 4100 Seraing (BE); CARDOL, Pierre, 4800 Verviers (BE)
(74) Mandataire: ABYOO
(86) Numéro de dépôt international: PCT/EP2017/053651
(87) Numéro de publication internationale: WO 2017/144367

(56) Documents cités:
- US-A1- 2006 151 332
- US-A1- 2013 015 076
- US-A1- 2013 256 152
- US-A1- 2015 240 368
- US-A1- 2015 321 929
- HANNAH S. SHAFAAT ET AL: "[NiFe] hydrogenases: A common active site for hydrogen metabolism under diverse conditions", BIOCHIMICA ET BIOPHYSICA ACTA. BIOENERGETICS., vol. 1827, no. 8-9, 8 février 2013 (2013-02-08), pages 986-1002, XP055311764, NL ISSN: 0005-2728, DOI: 10.1016/j.bbabio.2013.01.015
- Chelsea L Mcintosh ET AL: "The [NiFe]-Hydrogenase of the Cyanobacterium Synechocystis sp. PCC 6803 Works Bidirectionally with a Bias to H 2 Production", J. Am. Chem. Soc, vol. 133, 15 June 2011 (2011-06-15), pages 11308-11319, XP055708402, DOI: 10.1021/ja203376y
- Kylie A. Vincent ET AL: "Electricity from low-level H2 in still air ? an ultimate test for an oxygen tolerant hydrogenase", Chemical Communications, no. 48, 1 January 2006 (2006-01-01), page 5033, XP055708316, ISSN: 1359-7345, DOI: 10.1039/b614272a

## Description

La présente invention se rapporte à un dispositif de dissociation d'une phase aqueuse pour produire de l'hydrogène gazeux, ledit dispositif comprenant :
- une première zone comprenant ladite phase aqueuse,
- un moyen de capture d'électrons,
- un moyen de réduction de protons, et
- une source énergétique électrique agencée pour appliquer un potentiel énergétique électrique entre ledit moyen de capture d'électrons et ledit moyen de réduction de protons pour produire, via une réaction d'oxydation de ladite phase aqueuse au niveau dudit moyen de capture d'électrons, de l'oxygène gazeux, des électrons et des protons,
ledit moyen de réduction de protons étant agencé pour réaliser une réaction de réduction desdits protons par lesdits électrons afin de produire de l'hydrogène gazeux et étant une interface échangeuse de protons constituant une séparation entre ladite première zone comprenant ladite phase aqueuse et une deuxième zone non aqueuse, ladite séparation étant une séparation ne laissant pas passer ladite phase aqueuse mais laissant passer les protons vers ladite deuxième zone non aqueuse,
ladite interface échangeuse de protons présentant :
- une face frontale située dans ladite première zone comprenant ladite phase aqueuse et étant orientée vers ledit moyen de capture d'électrons, et
- une face dorsale située dans ladite deuxième zone non aqueuse et comprenant au moins un catalyseur et/ou au moins un système catalytique.

Par les termes « phase aqueuse », on entend, au sens de la présente invention, une phase contenant uniquement de l'eau ou une phase contenant de l'eau et au moins un additif, par exemple un électrolyte (tampon) ou un médiateur de transport d'électrons ou un accepteur d'électrons.

Le développement de tels dispositifs a pour objectif premier de pouvoir fournir un vecteur d'énergie stockable au départ d'une source énergétique, en particulier au départ d'une source énergétique électrique, ceci en essayant de minimiser au mieux l'apport énergétique, en particulier l'apport énergétique électrique, permettant le fonctionnement du dispositif.

Un tel dispositif est connu de l'état de la technique (voir par exemple la demande de brevet US 2015/240368) est utilisé pour obtenir, au départ d'une source énergétique électrique et d'eau, de l'hydrogène sous forme de gaz (H_{2 gaz}). Plus particulièrement, un tel dispositif permet l'obtention d'hydrogène gazeux par réalisation (1) d'une réaction d'oxydation d'une phase (solution) aqueuse donnant lieu à une libération d'oxygène gazeux (O_{2 gaz}), d'électrons (e⁻) et de protons (H⁺) et (2) d'une réaction de réduction desdits protons (H⁺) par lesdits électrons (e⁻), cette réaction de réduction produisant de l'hydrogène sous forme de gaz (H₂ gaz).

Dans une telle cellule ou dispositif, l'oxydation de la phase (solution) aqueuse s'effectue typiquement au niveau d'une anode comprenant une interface électrochimique (par exemple en carbone) qui assure une dissociation des molécules d'eau sous l'effet de l'énergie électrique apportée au dispositif via la source d'énergie électrique, c'est-à-dire sous l'effet de l'application d'un potentiel électrique entre ledit moyen de capture d'électrons et ledit moyen de réduction de protons.

Cette dissociation des molécules d'eau s'effectue selon la première réaction suivante : 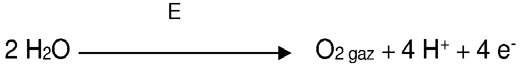 où E correspond à l'énergie électrique appliquée au dispositif, H₂O est l'eau, H⁺ représente un proton et e⁻ représente un électron.

Selon cette première réaction, un apport suffisant en énergie électrique permet de produire, par dissociation de l'eau, de l'oxygène (O_{2 gaz}), des protons (H⁺) et des électrons (e⁻). Eventuellement, les électrons ainsi obtenus par dissociation de l'eau vont être pris en charge, dans la solution aqueuse, par un médiateur (accepteur) d'électrons (par exemple du 2,5-dichloro-1,4-benzoquinone ou DCBQ) qui va leur faire gagner un moyen de capture d'électrons, c'est-à-dire une anode. Dès lors que l'anode est connectée électriquement à la cathode (c'est-à-dire à un moyen de réduction de protons) composée d'une interface électrochimique (par exemple en platine et/ou comprenant des enzymes de type hydrogénase), les électrons vont gagner cette dernière. De leur côté, les protons vont gagner, par diffusion au travers de la phase aqueuse, la cathode également. C'est au niveau de cette dernière que, finalement, les électrons et les protons obtenus par dissociation de l'eau sous l'effet du potentiel électrique appliqué, vont former de l'hydrogène gazeux au travers d'une réaction de réduction des protons selon la deuxième réaction suivante :

2 H⁺ + 2 e⁻ → H_{2 gaz}

De l'hydrogène gazeux (H_{2 gaz}) est donc finalement obtenu et peut être extrait du dispositif en vue par exemple de son stockage.

Un tel dispositif est donc classiquement composé de deux électrodes, par exemple sous forme de grilles en platine et/ou en carbone, baignant dans une même solution aqueuse et reliées entre elles, par exemple par l'intermédiaire d'un potentiostat. Avec un tel dispositif de dissociation de l'eau actuellement connu où les électrodes se présentent par exemple sous forme de grilles généralement en platine et/ou en carbone, ce potentiostat (ou tout autre appareillage adéquat) doit impérativement fournir un potentiel énergétique important et non négligeable afin d'assurer une production d'hydrogène gazeux (H_{2 gaz}). Il apparait en effet, qu'avec les dispositifs actuellement connus de l'état de la technique, un tel potentiel énergétique important est indispensablement requis pour permettre une production d'hydrogène gazeux. Plus particulièrement, un tel potentiel énergétique est indispensable pour permettre un transfert des électrons produits au niveau de l'anode jusqu'à la cathode et donc pour permettre, au niveau de cette dernière, la réduction des protons H⁺ en hydrogène gazeux, ceci par les électrons e⁻.

Il s'avère en effet que les électrons issus de l'eau sont confrontés à une cascade de transmission et à diverses interfaces à franchir avant de pouvoir d'abord gagner l'anode puis ensuite la cathode, chacune de ces transmissions et chacune de ces interfaces consommant une certaine énergie des électrons.

De tout ceci, il ressort donc que, malheureusement, pour les dispositifs actuels, il est indispensable de fournir un potentiel énergétique important pour que les électrons puissent disposer d'une énergie suffisante afin de surmonter toutes ces transmissions et toutes ces interfaces pour gagner la cathode (le moyen de réduction des protons) et y assurer la réduction des protons en hydrogène gazeux. Il en résulte que le bilan énergétique global et le rendement énergétique global de ces dispositifs ne sont pas optimaux.

Il existe donc un réel besoin de pouvoir procurer un dispositif pour la production d'hydrogène gazeux qui puisse minimiser l'apport énergétique, par exemple l'apport énergétique électrique, nécessaire ou qui puisse du moins réduire considérablement le potentiel énergétique requis pour obtenir une production d'hydrogène gazeux.

Pour résoudre ce problème, il est prévu suivant l'invention, un dispositif de dissociation d'une phase aqueuse pour produire de l'hydrogène gazeux tel qu'indiqué au début, ledit dispositif étant caractérisé en ce que ledit au moins un catalyseur et/ou ledit au moins un système catalytique comprend des enzymes de type hydrogénases.

Par les termes « catalyseur » ou « système catalytique », on entend, au sens de la présente invention, tout agent ou tout ensemble d'agents permettant de catalyser une réaction d'oxydation et/ou de réduction.

Par les termes « seule ladite face dorsale de ladite interface échangeuse de protons comprenant au moins un catalyseur et/ou au moins un système catalytique », on entend, au sens de la présente invention, qu'uniquement la face dorsale de l'interface échangeuse de protons est dopée avec au moins un catalyseur et/ou avec au moins un système catalytique.

Selon l'invention, l'interface (comme par exemple une membrane) échangeuse de protons, dénommée également membrane à électrolyte polymère (PEM), est une interface permettant la conduction protonique tout en ne laissant pas passer les gaz tels que le dioxygène ou le dihydrogène.

De façon surprenante, il a été montré, dans le cadre de la présente invention, qu'un tel dispositif dont le moyen de réduction de protons est une interface échangeuse de protons dont seule face dorsale comprend au moins un catalyseur et/ou au moins un système catalytique, permet de minimiser considérablement le potentiel énergétique, par exemple le potentiel électrique à appliquer au dispositif. En effet, il a été déterminé que le dispositif suivant l'invention permet de réduire de près d'un volt le potentiel énergétique à fournir au dispositif pour produire de l'hydrogène gazeux.

Plus particulièrement, il a été déterminé, dans le cadre de la présente invention, qu'un potentiel énergétique moindre doit être appliqué lorsque les protons gagnant l'interface échangeuse de protons sont pris en charge au niveau de la face frontale de cette dernière puis transportés au travers de cette interface échangeuse de protons de telle façon à gagner sa face dorsale où les protons sont directement réduits dès lors que seule la face dorsale de cette interface échangeuse de protons comprend au moins un catalyseur et/ou au moins un système catalytique.

De préférence, selon l'invention, ledit moyen de capture d'électrons comprend ou non au moins un catalyseur et/ou au moins un système catalytique.

Avantageusement, selon l'invention, ledit moyen de capture d'électrons est une interface échangeuse de protons ou une grille en carbone.

Selon l'invention, ledit au moins un catalyseur et/ou ledit au moins un système catalytique comprend des enzymes de type hydrogénase et/ou des particules de platine. Il a été déterminé que, outre un coût bien moindre par rapport au platine, des enzymes de type hydrogénase permettent de prendre en charge un nombre plus élevé d'électrons par unité de temps (par seconde) mais aussi de réduire quelque peu le potentiel énergétique requis par le dispositif afin de produire de l'hydrogène gazeux. Les enzymes de type hydrogénase mentionnées plus haut peuvent être éventuellement obtenues par voie de synthèse plutôt que d'être extraites au départ d'organismes naturels.

Selon l'invention, ladite interface échangeuse de protons constitue une séparation entre ladite première zone comprenant ladite phase aqueuse et une deuxième zone non aqueuse.

Par les termes « deuxième zone non aqueuse », on entend par exemple, au sens de la présente invention, une zone comprenant une phase solide ou une phase liquide non aqueuse ou une zone sans phase liquide.

Selon l'invention, ladite séparation est une séparation ne laissant pas passer ladite phase aqueuse ni les gaz (en particulier l'O₂) tout en laissant passer les protons. Une telle séparation suivant l'invention permet, dans un mode de réalisation, d'assurer que la réaction de réduction ne s'effectue qu'au niveau d'une zone non aqueuse. Dans ce cas, la séparation (l'interface, par exemple sous la forme d'une membrane) présentant une face frontale et une face dorsale, il est prévu, selon l'invention, que la face frontale soit en contact avec la phase aqueuse ou en contact direct avec l'anode et donc orientée du côté de la première zone aqueuse tandis qu'il est prévu que la face dorsale dopée avec un catalyseur (par exemple avec du carbone et/ou du platine) soit orientée du côté de la deuxième zone non aqueuse, la réaction de réduction des protons par les électrons afin d'obtenir de l'hydrogène gazeux s'effectuant uniquement au niveau de la face dorsale de l'interface (membrane) et donc uniquement du côté de la zone non aqueuse. Ceci est possible dès lors que, comme indiqué plus haut, la séparation (membrane) ne laisse pas passer la phase aqueuse mais laisse passer les protons vers la deuxième zone non aqueuse, les protons étant réduits uniquement au niveau de la face dorsale de cette séparation (interface) et donc au niveau de la deuxième zone non aqueuse. Dans le cadre de la présente invention, il a été déterminé qu'une réduction des protons au niveau d'une zone non aqueuse permet d'optimiser l'intensité (la grandeur / importance du courant) pour un même potentiel énergétique appliqué au système.

Préférentiellement, selon l'invention, un moyen de contact est présent au niveau dudit moyen de capture d'électrons et/ou au niveau dudit moyen de réduction des protons. Par exemple, un tissu en carbone est prévu pour assurer un contact électrique optimal entre ledit moyen de capture d'électrons et ledit moyen de réduction des protons, ceci par l'intermédiaire par exemple d'un potentiostat.

De préférence, selon l'invention, ladite phase aqueuse est une phase contenant uniquement de l'eau ou une phase contenant de l'eau et au moins un additif, par exemple un électrolyte ou un médiateur de transport d'électrons ou un accepteur d'électrons.

De préférence, selon l'invention, ladite phase aqueuse comprend en outre un médiateur de transport d'électrons ou un accepteur d'électrons. Eventuellement, ledit médiateur de transport d'électrons ou ledit accepteur d'électrons est sous forme de nanotubes de carbone (Carbon Nano-Tube) ou de ferricyanure.

Avantageusement, selon l'invention, ladite phase aqueuse présente une valeur de pH comprise entre 0,1 et 10, de préférence une valeur de pH comprise entre 6 et 7.

De préférence, le dispositif de dissociation d'une phase aqueuse pour produire de l'hydrogène gazeux selon l'invention comprend en outre un dispositif additionnel de récupération et d'évacuation de gaz. Par exemple, ce dispositif peut se présenter sous la forme d'un dispositif assurant un flux azote au niveau de ladite deuxième zone non aqueuse, ceci afin de récupérer et d'évacuer les gaz présents dans ladite deuxième zone non aqueuse, notamment afin de récupérer et d'évacuer l'hydrogène gazeux produit par réduction des protons au niveau de cette deuxième zone non aqueuse.

De préférence, le dispositif de dissociation d'une phase aqueuse pour produire de l'hydrogène gazeux selon l'invention comprend en outre un dispositif additionnel de détection d'hydrogène gazeux.

D'autres formes de réalisation du dispositif suivant l'invention sont indiquées dans les revendications annexées.

L'invention a aussi pour objet un procédé de production d'hydrogène gazeux avec un dispositif selon l'invention, au départ d'une phase aqueuse et d'une source énergétique, ledit procédé comprenant les étapes suivantes :
- une application d'un potentiel énergétique entre un moyen de capture d'électrons et une interface échangeuse de protons présentant une face frontale orientée vers ledit moyen de capture d'électrons et présentant une face dorsale comprenant au moins un catalyseur et/ou au moins un système catalytique, pour produire, au niveau dudit moyen de capture d'électrons, via une réaction d'oxydation de ladite phase aqueuse présente dans une première zone, de l'oxygène gazeux, des électrons et des protons, et
- une capture desdits protons, au niveau de ladite interface échangeuse de protons présentant une face frontale orientée vers ledit moyen de capture d'électrons et présentant une face dorsale comprenant au moins un catalyseur et/ou au moins un système catalytique, afin que lesdits protons soient réduits en hydrogène gazeux via une réaction de réduction desdits protons par lesdits électrons au niveau de ladite face dorsale de ladite interface échangeuse de protons.

D'autres formes de réalisation du procédé suivant l'invention sont indiquées dans les revendications annexées.

L'invention a aussi pour objet une utilisation d'un dispositif selon l'invention pour produire de l'hydrogène gazeux au départ d'une phase aqueuse et d'une source énergétique.

D'autres formes d'utilisation d'un dispositif selon l'invention sont indiquées dans les revendications annexées.

La figure 1 est une vue schématique éclatée d'un dispositif pour produire de l'hydrogène gazeux au départ d'une phase aqueuse et d'une source énergétique, en l'occurrence au départ d'une phase aqueuse et d'une source énergétique électrique.

La figure 2 est une vue schématique éclatée d'un autre dispositif pour produire de l'hydrogène gazeux au départ d'une phase aqueuse et d'une source énergétique, en l'occurrence au départ d'une phase aqueuse et d'une source énergétique électrique.

La figure 3 est un graphique reprenant les résultats enregistrés au travers de mesures d'ampéro-multivoltage (de 0,1V à 0,9V) pour les dispositifs (comprenant une interface échangeuse de protons dopée - trait continu) illustrés aux figures 1 et 2, en comparaison avec un dispositif de l'état de la technique (comprenant une interface échangeuse de protons non dopée et une grille de platine - trait interrompu).

La figure 4 est un graphique comparant les résultats enregistrés au travers de mesures d'ampéro-multivoltage (de 0,1V à 0,9V) pour deux dispositifs différents illustrés aux figures 1 (trait interrompu) ou 2 (trait continu) et comprenant une interface échangeuse de protons dopée.

La figure 5 est une vue schématique éclatée d'un autre dispositif pour produire de l'hydrogène gazeux au départ d'une phase aqueuse et d'une source énergétique, en l'occurrence au départ d'une phase aqueuse et d'une source énergétique électrique.

Sur les figures, les éléments identiques ou analogues portent les mêmes références.

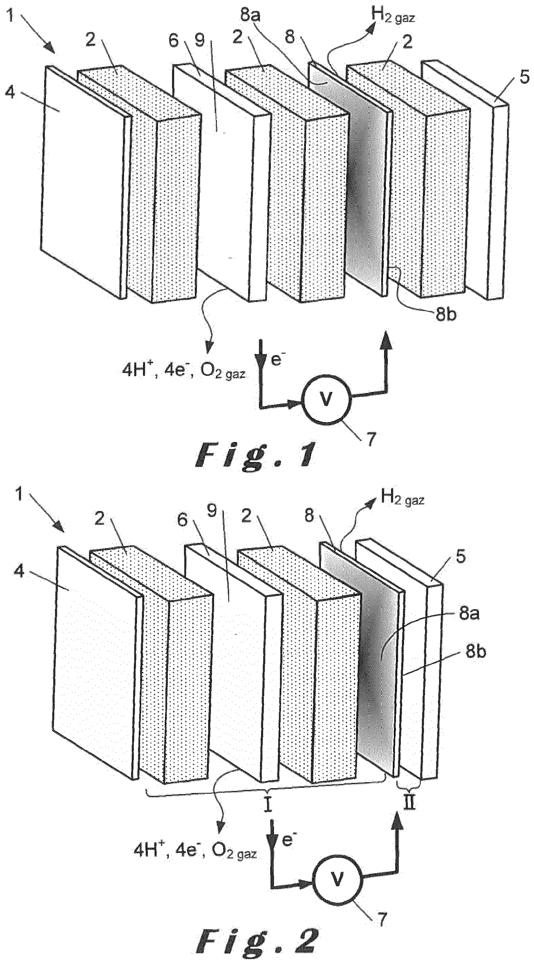

La figure 1 illustre un dispositif 1 pour produire de l'hydrogène gazeux (H_{2 gaz}) au départ d'une phase aqueuse 2 et d'une source énergétique électrique 7. Ce dispositif 1 comprend une paroi frontale 4 et une paroi dorsale 5, par exemple des parois 4, 5 en un polymère adéquat. Bien entendu, le dispositif 1 comprend également des parois supérieure et inférieure et des parois latérales de telle façon à former un dispositif (générateur) étanche.

Dans la phase aqueuse 2 baigne une anode 6 (par exemple une anode 6 en carbone) reliée par l'intermédiaire d'un potentiostat 7 (source énergétique électrique) à une interface sous la forme d'une membrane 8 échangeuse de protons présentant une face frontale 8a orientée vers ledit moyen de capture d'électrons 6 et présentant une face dorsale 8b comprenant au moins un catalyseur et/ou au moins un système catalytique, la membrane (interface) 8 baignant également dans la même phase aqueuse 2. Lors d'une application d'un potentiel électrique entre l'anode 6 et la membrane (interface) 8, l'anode 6 va être le siège d'une réaction d'oxydation de la phase aqueuse 2 afin d'y produire de l'oxygène gazeux (O_{2 gaz}), des électrons (4 e⁻) et des protons libres (4 H⁺). Plus particulièrement, la membrane (interface) 8 présente une face frontale 8a en contact avec la phase aqueuse 2 et présente également une face dorsale 8b également en contact avec la phase aqueuse et étant dopée avec du platine de telle façon à pouvoir jouer le rôle de cathode. La réaction de réduction des protons (H⁺) par les électrons (e⁻) afin d'obtenir de l'hydrogène gazeux (H_{2 gaz}) s'effectue uniquement au niveau de la face dorsale 8b de la membrane (interface) 8.

Préférentiellement, la phase aqueuse 2 comprend un médiateur de transport d'électrons ou un accepteur d'électrons (par exemple de type DCBQ) prenant en charge ces derniers pour leur faire gagner l'anode 6 de telle sorte que, puisque cette dernière est connectée électriquement à la membrane (interface) 8 par l'intermédiaire d'un potentiostat 7, les électrons puissent gagner la membrane (interface) 8 et y rejoindre les protons, lesquels auront également gagné la membrane (interface) 8 au travers de la phase aqueuse 2. Se réalise alors, au niveau de la face dorsale de la membrane (cathode) 8, une réaction de réduction des protons H⁺ par les électrons e⁻ de telle sorte à former de l'hydrogène gazeux (H_{2 gaz}). Eventuellement, un tissu en carbone (Gaz Diffusion Layer - GDL) est placé sur la face dorsale 8b (orientée vers la paroi 5) de la membrane (interface) 8 (dopée et jouant le rôle de cathode) pour former une couche de contact électrique entre la membrane (cathode) 8 et le potentiostat 7.

La figure 2 illustre un dispositif 1 pour produire de l'hydrogène gazeux (H_{2 gaz}) au départ d'une phase aqueuse 2 et d'une source d'énergie électrique 7. Ce dispositif 1 tel qu'illustré à la figure 2 présente une première zone I aqueuse et une deuxième zone II non aqueuse. Selon ce mode de réalisation, une membrane (interface) 8 de type PEM dopée avec du carbone et du platine constitue une séparation entre la première zone I aqueuse et la deuxième zone II non aqueuse. Le dopage de la membrane (interface) 8 avec du carbone et du platine permet à celle-ci de jouer le rôle de cathode. Cette séparation sous forme d'une membrane (interface) 8 ne laisse pas passer la phase aqueuse 2 ni les gaz mais laisse passer les protons (H⁺) depuis ladite zone I aqueuse vers ladite zone II non aqueuse. Plus particulièrement, cette membrane 8 (séparation) présente une face frontale 8a en contact avec la phase aqueuse 2 et donc orientée du côté de la première zone I aqueuse. Cette membrane (séparation) présente également une face dorsale 8b dopée avec du platine de telle façon à pouvoir jouer le rôle de cathode, cette face dorsale 8b étant orientée du côté de la deuxième zone II non aqueuse. Selon ce mode de réalisation, la réaction de réduction des protons (H⁺) par les électrons (e⁻) afin d'obtenir de l'hydrogène gazeux (H_{2 gaz}) s'effectue uniquement au niveau de la face dorsale 8b de la séparation (membrane) et donc uniquement du côté de la zone II non aqueuse. Eventuellement, un tissu en carbone (Gaz Diffusion Layer - GDL) est placé sur la face dorsale 8b (orientée vers la paroi 5) de la membrane (interface) 8 (dopée et jouant le rôle de cathode) pour former une couche de contact électrique entre la membrane (cathode) 8 et le potentiostat 7.

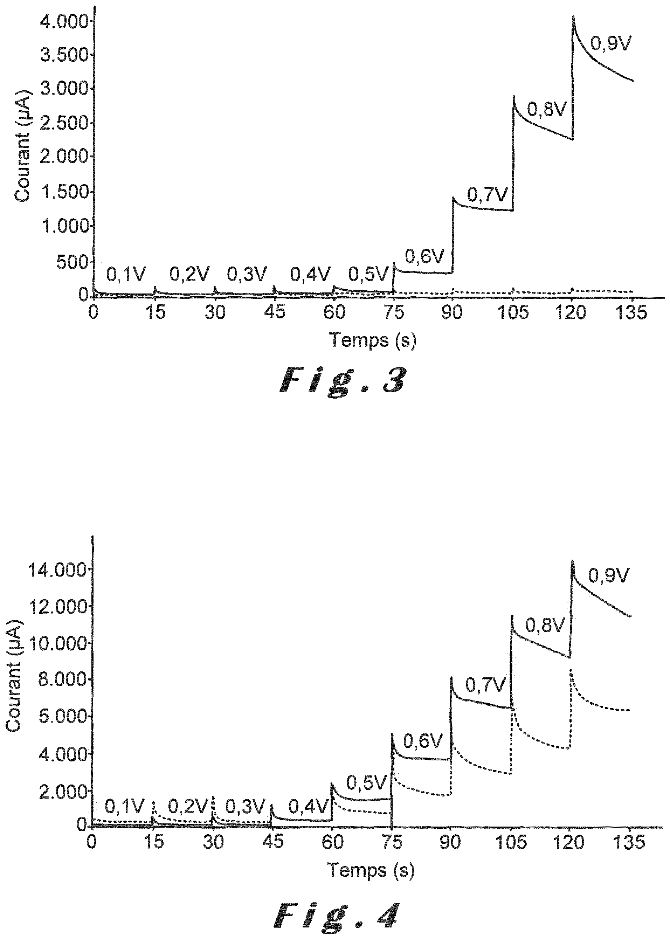

La figure 3 reprend les résultats enregistrés au travers de mesures d'ampéro-multivoltage (de 0,1V à 0,9V) pour les dispositifs illustrés aux figures 1 et 2 (courbe en trait continu - dispositifs comprenant une membrane / interface dopée avec du platine), ces résultats étant comparés à ceux mesurés dans les mêmes conditions pour un dispositif de l'état de la technique comprenant une membrane non dopée et une grille en platine (courbe en trait interrompu - état de la technique). Comme on peut le constater, pour ces dispositifs , un courant est mesuré entre l'anode et la membrane (interface) (cathode) dès l'application d'un potentiel de 0,6V tandis que, pour un dispositif selon l'état de la technique, aucun courant n'est mesuré entre l'anode et la cathode (grille de platine), ceci même lors d'une application de potentiels supérieurs (par exemple de 0,9V). Par ailleurs, pour ces dispositifs, le courant mesuré augmente considérablement pour des potentiels appliqués de 0,7V, de 0,8V et 0,9V. Il ressort donc de ces essais comparatifs que ces dispositifs dont le moyen de capture de protons est une interface échangeuse de protons, dont seule la face dorsale comprend au moins un catalyseur et/ou au moins un système catalytique, permet d'obtenir un rendement énergétique amélioré par rapport au dispositif connu de l'état de la technique.

Pour ces essais comparatifs, la phase aqueuse 2 comprenant du DCBQ (0,75 mM) et un tampon (NaCI 50 mM, MES 20 mM, MgCI 2mM). En outre, pour le mode de réalisation illustré à la figure 2, était présent lors des essais, un dispositif additionnel de récupération et d'évacuation de gaz présents dans la zone non aqueuse.

La figure 4 est un graphique comparant les résultats enregistrés au travers de mesures d'ampéro-multivoltage (de 0,1V à 0,9V) pour les dispositifs illustrés aux figures 1 (trait interrompu) ou 2 (trait continu). Pour chacun de ces modes de réalisation illustrés aux figures 1 ou 2, une interface échangeuse de protons (membrane PEM) dopée avec du platine joue le rôle de cathode. Comme il ressort de ce graphique, l'intensité du courant (µA) est plus importante, pour un même potentiel appliqué au système, lorsque ce dernier comprend une interface échangeuse de protons séparant une première zone aqueuse d'une deuxième zone non aqueuse (mode de réalisation de la figure 2). Sur ce graphique, ce mode de réalisation de la figure 2 est comparé à un dispositif selon la figure 1 où n'est pas présente une zone non aqueuse. Comme illustré, le dispositif illustré à la figure 2 présentant une zone non aqueuse où a lieu la réaction de réduction des protons permet d'optimiser l'intensité de courant, ce qui implique une production d'H_{2 gaz} plus importante, ceci pour une application d'un potentiel énergétique identique et prédéterminé, en comparaison avec un dispositif ne comprenant pas de zone aqueuse.

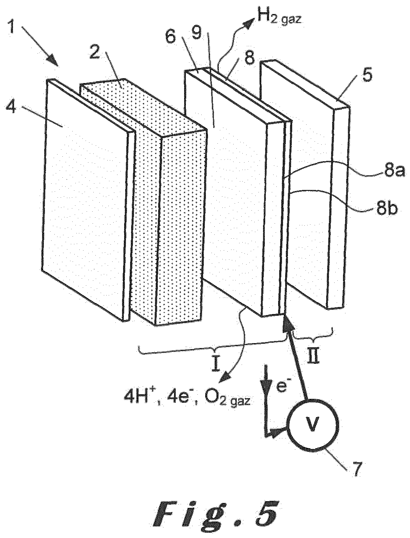

La figure 5 est une vue schématique d'un autre dispositif pour produire de l'hydrogène gazeux (H_{2 gaz}) au départ d'une phase aqueuse 2 et d'une source énergétique électrique 7. Ce dispositif 1 est identique à celui illustré à la figure 2, à la différence que la membrane (interface) 8 est accolée à la face dorsale de l'anode 6. La membrane (interface) 8 constitue une séparation entre la première zone I aqueuse et la deuxième zone II non aqueuse et est dopée avec du carbone et du platine de telle façon à pouvoir jouer le rôle de cathode. Cette séparation sous forme d'une membrane 8 ne laisse pas passer la phase aqueuse 2 ni les gaz (en particulier l'O₂) mais laisse passer les protons (H⁺). Plus particulièrement, cette membrane (interface) 8 présente une face frontale 8a en contact direct avec l'anode 6 (par accolement contre la face dorsale de l'anode 6), cette face frontale 8a étant orientée du côté de la première zone I aqueuse. Cette membrane (interface) 8 présente également une face dorsale 8b dopée avec du platine de telle façon à pouvoir jouer le rôle de cathode, cette face dorsale 8b étant orientée du côté de la deuxième zone II non aqueuse. La réaction de réduction des protons (H⁺) par les électrons (e⁻) afin d'obtenir de l'hydrogène gazeux (H_{2 gaz}) s'effectue uniquement dans cette zone II non aqueuse au niveau de la face dorsale de la membrane 8. Eventuellement, un tissu en carbone (Gaz Diffusion Layer - GDL) est placé sur la face dorsale (orientée vers la paroi 5) de la membrane (interface ou séparation) 8 (dopée et jouant le rôle de cathode) pour former une couche de contact électrique entre la membrane (cathode) 8 et le potentiostat 7.

Selon ce dispositif illustré à la figure 5, la réaction de réduction s'effectue dans la zone II non aqueuse mais, en plus, les protons obtenus dans la phase aqueuse via la réaction d'oxydation sont directement pris en charge par la membrane (interface) 8 sans avoir à traverser une phase aqueuse comme c'est le cas pour le dispositif illustré à la figure 2. Ici, les protons ne se retrouvent pas dans une phase aqueuse à nouveau : ils sont pris en charge directement par la membrane (interface) 8 qui joue le rôle de cathode dans la zone II non aqueuse.

Il est bien entendu que la présente invention n'est en aucune façon limitée aux formes de réalisations décrites ci-dessus et que bien des modifications peuvent y être apportées sans sortir de l'étendue de la protection définie par les revendications annexées.

## Revendications

1. Dispositif de dissociation d'une phase aqueuse pour produire de l'hydrogène gazeux, ledit dispositif comprenant :
- une première zone comprenant ladite phase aqueuse,
- un moyen de capture d'électrons,
- un moyen de réduction de protons, et
- une source énergétique électrique agencée pour appliquer un potentiel énergétique électrique entre ledit moyen de capture d'électrons et ledit moyen de réduction de protons pour produire, via une réaction d'oxydation de ladite phase aqueuse au niveau dudit moyen de capture d'électrons, de l'oxygène gazeux, des électrons et des protons,
ledit moyen de réduction de protons étant agencé pour réaliser une réaction de réduction desdits protons par lesdits électrons afin de produire de l'hydrogène gazeux et étant une interface échangeuse de protons constituant une séparation entre ladite première zone comprenant ladite phase aqueuse et une deuxième zone non aqueuse, ladite séparation étant une séparation ne laissant pas passer ladite phase aqueuse mais laissant passer les protons vers ladite deuxième zone non aqueuse,
ladite interface échangeuse de protons présentant :
- une face frontale située dans ladite première zone comprenant ladite phase aqueuse et étant orientée vers ledit moyen de capture d'électrons, et
- une face dorsale située dans ladite deuxième zone non aqueuse et comprenant au moins un catalyseur et/ou au moins un système catalytique,
ledit dispositif étant **caractérisé en ce que** ledit au moins un catalyseur et/ou ledit au moins un système catalytique comprend des enzymes de type hydrogénases.

2. Dispositif de dissociation d'une phase aqueuse pour produire de l'hydrogène gazeux selon la revendication 1, **caractérisé en ce que** ledit moyen de capture d'électrons comprend ou non au moins un catalyseur et/ou au moins un système catalytique.

3. Dispositif de dissociation d'une phase aqueuse pour produire de l'hydrogène gazeux selon la revendication 1 ou 2, **caractérisé en ce que** ledit moyen de capture d'électrons est une interface échangeuse de protons ou une grille en carbone.

4. Dispositif de dissociation d'une phase aqueuse pour produire de l'hydrogène gazeux selon l'une quelconque des revendications 1 à 3, **caractérisé en ce que** ledit au moins un catalyseur et/ou ledit au moins un système catalytique compris par ladite face dorsale de ladite interface échangeuse de protons comprend des particules de platine.

5. Dispositif photo-catalytique de dissociation d'une phase aqueuse pour produire de l'hydrogène gazeux selon l'une quelconque des revendications 1 à 4, **caractérisé en ce que** ladite séparation est une séparation ne laissant pas passer ladite phase aqueuse ni les gaz tout en laissant passer les protons.

6. Dispositif de dissociation d'une phase aqueuse pour produire de l'hydrogène gazeux selon l'une quelconque des revendications 1 à 5, **caractérisé en ce que** ladite phase aqueuse est une phase contenant uniquement de l'eau ou une phase contenant de l'eau et au moins un additif, par exemple un électrolyte ou un médiateur de transport d'électrons ou un accepteur d'électrons.

7. Dispositif de dissociation d'une phase aqueuse pour produire de l'hydrogène gazeux selon l'une quelconque des revendications 1 à 6, **caractérisé en ce que** ladite phase aqueuse comprend en outre un médiateur de transport d'électrons ou un accepteur d'électrons.

8. Dispositif de dissociation d'une phase aqueuse pour produire de l'hydrogène gazeux selon l'une quelconque des revendications 1 à 7, **caractérisé en ce que** ladite phase aqueuse présente une valeur de pH comprise entre 0,1 et 10, de préférence une valeur de pH comprise entre 6 et 7.

9. Dispositif de dissociation d'une phase aqueuse pour produire de l'hydrogène gazeux selon l'une quelconque des revendications 1 à 8, **caractérisé en ce qu'**il comprend en outre un dispositif additionnel de récupération et d'évacuation de gaz.

10. Dispositif de dissociation d'une phase aqueuse pour produire de l'hydrogène gazeux selon l'une quelconque des revendications 1 à 9, **caractérisé en ce qu'**il comprend en outre un dispositif additionnel de détection d'hydrogène gazeux.

11. Procédé de production d'hydrogène gazeux avec un dispositif selon l'une quelconque des revendications 1 à 10, au départ d'une phase aqueuse et d'une source énergétique électrique, ledit procédé comprenant les étapes suivantes :
- une application d'un potentiel énergétique électrique entre un moyen de capture d'électrons et une interface échangeuse de protons présentant une face frontale orientée vers ledit moyen de capture d'électrons et présentant une face dorsale comprenant au moins un catalyseur et/ou au moins un système catalytique, pour produire, au niveau dudit moyen de capture d'électrons, via une réaction d'oxydation de ladite phase aqueuse présente dans une première zone, de l'oxygène gazeux, des électrons et des protons, et
- une capture desdits protons, au niveau de ladite interface échangeuse de protons présentant une face frontale orientée vers ledit moyen de capture d'électrons et présentant une face dorsale comprenant au moins un catalyseur et/ou au moins un système catalytique, afin que lesdits protons soient réduits en hydrogène gazeux via une réaction de réduction desdits protons par lesdits électrons au niveau de ladite face dorsale de ladite interface échangeuse de protons.

12. Utilisation d'un dispositif selon l'une quelconque des revendications 1 à 10 pour produire de l'hydrogène gazeux au départ d'une phase aqueuse et d'une source énergétique électrique.

## Patentansprüche

1. Dissoziationsvorrichtung einer wässerigen Phase zum Produzieren von gasförmigem Wasserstoff, wobei die genannte Vorrichtung umfasst:
- einen ersten Bereich, der die genannte wässerige Phase umfasst,
- ein Elektroneneinfangmittel,
- ein Protonenreduktionsmittel und
- eine elektrische Energiequelle, die angeordnet ist, um ein elektrisches Energiepotential zwischen dem genannten Elektroneneinfangmittel und dem genannten Protonenreduktionsmittel anzuwenden, um über eine Oxidationsreaktion der genannten wässrigen Phase an dem genannten Elektroneneinfangmittel gasförmigen Sauerstoff, Elektronen und Protonen zu produzieren,
wobei das genannte Protonenreduktionsmittel angeordnet ist, um eine Reduktionsreaktion der genannten Protonen durch die genannten Elektronen zu realisieren, um gasförmigen Wasserstoff zu produzieren und eine Protonenaustauschplattform zu sein, die eine Trennung zwischen dem genannten ersten Bereich, der die genannte wässrige Phase und einen zweiten, nicht wässrigen Bereich umfasst, bildet, wobei die genannte Trennung eine Trennung ist, die die genannte wässrige Phase nicht hindurchtreten lässt, sondern die Protonen zu dem genannten, zweiten, nicht wässrigen Bereich hindurchtreten lässt,
wobei die genannte Protonenaustauschplattform aufweist:
- eine Vorderseite, die in dem genannten ersten Bereich angeordnet ist, umfassend die genannte wässrige Phase, und die zu dem genannten Elektroneneinfangmittel ausgerichtet ist, und
- eine Rückseite, die in dem genannten zweiten, nicht wässrigen Bereich angeordnet ist und wenigstens einen Katalysator und / oder wenigstens ein katalytisches System umfasst,
wobei die genannte Vorrichtung **dadurch gekennzeichnet ist, dass** der genannte wenigstens eine Katalysator und / oder das genannte wenigstens eine katalytische System Enzyme vom Typ Hydrogenasen umfasst.

2. Dissoziationsvorrichtung einer wässerigen Phase zum Produzieren von gasförmigem Wasserstoff gemäß Anspruch 1, **dadurch gekennzeichnet, dass** das genannte Elektroneneinfangmittel wenigstens einen Katalysator und / oder wenigstens ein katalytisches System umfasst.

3. Dissoziationsvorrichtung einer wässerigen Phase zum Produzieren von gasförmigem Wasserstoff gemäß Anspruch 1 oder 2, **dadurch gekennzeichnet, dass** das genannte Elektroneneinfangmittel eine Protonenaustauschschnittstelle oder ein Kohlenstoffgitter ist.

4. Dissoziationsvorrichtung einer wässerigen Phase zum Produzieren von gasförmigem Wasserstoff gemäß irgendeinem der Ansprüche 1 bis 3, **dadurch gekennzeichnet, dass** der genannte wenigstens eine Katalysator und / oder das genannte wenigstens eine katalytische System, das zwischen der genannten Rückseite und der genannten Protonenaustauschschnittstelle inbegriffen ist, Platinpartikel umfasst.

5. Photokatalytische Dissoziationsvorrichtung einer wässrigen Phase zum Produzieren von gasförmigem Wasserstoff gemäß irgendeinem der Ansprüche 1 bis 4, **dadurch gekennzeichnet, dass** die genannte Trennung eine Trennung ist, die weder die genannte wässrige Phase noch das Gas hindurchtreten lässt und dabei jedoch die Protonen hindurchtreten lässt.

6. Dissoziationsvorrichtung einer wässerigen Phase zum Produzieren von gasförmigem Wasserstoff gemäß irgendeinem der Ansprüche 1 bis 5, **dadurch gekennzeichnet, dass** die genannte wässrige Phase eine Phase, die ausschließlich Wasser enthält, oder eine Phase ist, die Wasser und wenigstens einen Zusatzstoff, z. B. ein Elektrolyt oder einen Transportvermittler von Elektronen oder einen Elektronenempfänger enthält.

7. Dissoziationsvorrichtung einer wässerigen Phase zum Produzieren von gasförmigem Wasserstoff gemäß irgendeinem der Ansprüche 1 bis 6, **dadurch gekennzeichnet, dass** die genannte wässrige Phase darüber hinaus einen Transportvermittler von Elektronen oder einen Elektronenempfänger umfasst.

8. Dissoziationsvorrichtung einer wässerigen Phase zum Produzieren von gasförmigem Wasserstoff gemäß irgendeinem der Ansprüche 1 bis 7, **dadurch gekennzeichnet, dass** die genannte wässrige Phase einen zwischen 0,1 und 10 inbegriffenen pH-Wert, bevorzugt einen zwischen 6 und 7 inbegriffenen pH-Wert aufweist.

9. Dissoziationsvorrichtung einer wässerigen Phase zum Produzieren von gasförmigem Wasserstoff gemäß irgendeinem der Ansprüche 1 bis 8, **dadurch gekennzeichnet, dass** sie darüber hinaus eine zusätzliche Gaswiedergewinnungs- und - austragsvorrichtung umfasst.

10. Dissoziationsvorrichtung einer wässerigen Phase zum Produzieren von gasförmigem Wasserstoff gemäß irgendeinem der Ansprüche 1 bis 9, **dadurch gekennzeichnet, dass** sie darüber hinaus eine zusätzliche Vorrichtung zur Detektion von gasförmigem Wasserstoff umfasst.

11. Produktionsverfahren von gasförmigem Wasserstoff mit einer Vorrichtung gemäß irgendeinem der Ansprüche 1 bis 10 zu Beginn einer wässrigen Phase und einer elektrischen Energiequelle, wobei das genannte Verfahren die folgenden Schritte umfasst:
- eine Anwendung eines elektrischen Energiepotentials zwischen einem Elektroneneinfangmittel und einer Protonenaustauschschnittstelle, die eine Vorderseite aufweist, die zu dem genannten Elektroneneinfangmittel ausgerichtet ist und eine Rückseite aufweist, umfassend wenigstens einen Katalysator und / oder wenigstens ein katalytisches System, um an dem genannten Elektroneneinfangmittel über eine Oxidationsreaktion der genannten wässrigen Phase, die in einem ersten Bereich vorhanden ist, gasförmigen Sauerstoff und Protonen zu produzieren und
- ein Einfangen der genannten Protonen an der genannten Protonenaustauschschnittstelle, die eine Vorderseite aufweist, die zu dem genannten Elektroneneinfangmittel ausgerichtet ist und eine Rückseite aufweist, die wenigstens einen Katalysator und / oder wenigsten ein katalytisches System aufweist, damit die genannten Protonen über eine Reduktionsreaktion der genannten Protonen durch die genannten Elektronen an der genannten Rückseite der genannten Protonenaustauschschnittstelle in gasförmigen Wasserstoff reduziert werden.

12. Verwendung einer Vorrichtung gemäß irgendeinem der Ansprüche 1 bis 10 zum Produzieren von gasförmigem Wasserstoff zu Beginn einer wässrigen Phase und einer elektrischen Energiequelle.

## Claims

1. Device for dissociating an aqueous phase to produce gaseous hydrogen, said device comprising:
- a first zone comprising said aqueous phase,
- a means of capturing electrons,
- a means of reducing protons, and
- an electrical energy source arranged to apply an electric energy potential between said electron capture means and said proton reduction means to produce, via an oxidation reaction of said aqueous phase at said electron capture means, gaseous oxygen, electrons and protons,
said proton reduction means being arranged to carry out a reduction reaction of said protons by said electrons in order to produce gaseous hydrogen and being a proton exchange interface constituting a separation between said first zone comprising said aqueous phase and a second non-aqueous zone, said separation being a separation not allowing said aqueous phase to pass but allowing protons to pass to said second non-aqueous zone,
said proton exchange interface exhibiting:
- a front face located in said first zone comprising said aqueous phase and facing towards said electron capture means, and
- a back face located in said second non-aqueous zone and comprising at least one catalyst and/or at least one catalytic system,
said device being **characterised in that** said at least one catalyst and/or said at least one catalytic system comprises enzymes of the hydrogenase type.

2. Device for dissociating an aqueous phase to produce gaseous hydrogen according to claim 1, **characterised in that** said electron capture means may or may not comprise at least one catalyst and/or at least one catalytic system.

3. Device for dissociating an aqueous phase to produce gaseous hydrogen according to claim 1 or 2, **characterised in that** said electron capture means is a proton exchange interface or a carbon mesh.

4. Device for dissociating an aqueous phase to produce gaseous hydrogen according to any one of claims 1 to 3, **characterised in that** said at least one catalyst and/or said at least one catalytic system comprised by said back face of said proton exchange interface comprises platinum particles .

5. Photo-catalytic device for dissociating an aqueous phase to produce gaseous hydrogen according to any one of claims 1 to 4, **characterised in that** said separation is a separation which does not allow said aqueous phase or gases to pass, while allowing the protons to pass.

6. Device for dissociating an aqueous phase to produce gaseous hydrogen according to any one of claims 1 to 5, **characterised in that** said aqueous phase is a phase containing only water or a phase containing water and at least one additive, for example an electrolyte or an electron transport mediator or an electron acceptor.

7. Device for dissociating an aqueous phase to produce gaseous hydrogen according to any one of claims 1 to 6, **characterised in that** said aqueous phase further comprises an electron transport mediator or an of electron acceptor.

8. Device for dissociating an aqueous phase to produce gaseous hydrogen according to any one of claims 1 to 7, **characterised in that** said aqueous phase has a pH value of between 0.1 and 10, preferably a pH value between 6 and 7.

9. Device for dissociating an aqueous phase to produce gaseous hydrogen according to any one of claims 1 to 8, **characterised in that** it further comprises an additional device for capturing and discharging gas.

10. Device for dissociating an aqueous phase to produce gaseous hydrogen according to any one of claims 1 to 9, **characterised in that** it further comprises an additional device for detecting gaseous hydrogen.

11. A method for producing gaseous hydrogen with a device according to any one of claims 1 to 10, starting from an aqueous phase and an electrical energy source, said method comprising the following steps:
- application of an electric energy potential between an electron capture means and a proton exchange interface having a front face facing said electron capture means and having a back face comprising at least one catalyst and/or at least one catalytic system, to produce, at said electron capture means, via an oxidation reaction of said aqueous phase present in a first zone, gaseous oxygen, electrons and protons, and
- a capture of said protons at said proton interface exchange having a front face facing said electron capture means and having a back face comprising at least one catalyst and/or at least one system catalyst, so that said protons are reduced to gaseous hydrogen via a reduction reaction of said protons by said electrons at said back face of said proton exchange interface.

12. Use of a device according to any one of claims 1 to 10 for producing gaseous hydrogen from an aqueous phase and an electrical energy source.
